(19) Europäisches Patentamt
European Patent Office
Office européen des brevets

(11) Publication number: **0 038 512**
**B1**

(12) **EUROPEAN PATENT SPECIFICATION**

(45) Date of publication of patent specification: **29.10.86**

(21) Application number: **81102849.7**

(22) Date of filing: **14.04.81**

(51) Int. Cl.⁴: **C 07 D 233/14,**
C 07 D 233/16,
C 07 D 233/18,
C 07 C 103/44, A 61 K 7/00,
A 61 K 47/00

# (54) Transdermal carrier materials.

(30) Priority: **17.04.80 CH 2973/80**

(43) Date of publication of application:
**28.10.81 Bulletin 81/43**

(45) Publication of the grant of the patent:
**29.10.86 Bulletin 86/44**

(84) Designated Contracting States:
**AT BE CH DE FR GB IT LI LU NL SE**

(56) References cited:
**GB-A-1 493 093**
**US-A-3 262 951**
**US-A-3 427 251**
**US-A-4 044 034**

(73) Proprietor: **Chemisches Institut Schäfer A.G.**
**Organ. Chem. Laboratorium für industrielle Forschung und Entwicklung**
**Sägestrasse 5**
**CH-4104 Oberwil (CH)**

(72) Inventor: **Schäfer, Werner**
**Auf der Wacht 33**
**CH-4104 Oberwil (CH)**
Inventor: **Schäfer, Rolf, Dr.**
**Grabenmattstrasse 37**
**CH-4133 Pratteln (CH)**
Inventor: **Schäfer, Doris, Dr.**
**Neue Blauenrainstrasse 9**
**CH-4411 Arisdorf (CH)**

(74) Representative: **Blum, Rudolf Emil Ernst et al**
**c/o E. Blum & Co Patentanwälte Vorderberg 11**
**CH-8044 Zürich (CH)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 038 512 B1**

Courier Press, Leamington Spa, England.

**Description**

BACKGROUND OF THE INVENTION

(a) Field of the Invention

This invention relates to complexes useful as carriers for pharmaceutical and cosmetic active materials. The compositions permit continued constant rate transdermal penetration of active materials, that is, through the cell membranes of skin and the gastrointestinal mucosa.

(b) State of the Art

Permeation of pharmaceutical and cosmetic active substances through body tissues when administered topically or orally is essential for effective treatment. Carrier materials for the active substances optimally should effect or permit penetration at a steady rate. Moreover, the carrier materials should not alter the cell membrane systems of the tissues or the proteins therein responsible for biochemical functions, such as susbtance transport.

Many surfactants or other strongly ionic materials which are used as penetration adjuvants, emulsifiers, wetting agents or the like in pharmaceutical or cosmetic compositions, as well as some strongly ionic active materials, alter the cell membrane, as by denaturing the membrane proteins, dissolving the lipid layer of the membrane or otherwise adversely affecting the membrane transport system. As a result, substance penetration is impeded or stopped and effective treatment is no longer achieved or excessively high doses are required to achieve the desired therapeutic effect. Such strongly ionic materials include also quaternary surfactants in which the anionic group is represented by chloride or bromide. An example of such a material which exhibits the above-mentioned disadvantages are quaternary ammonium compounds described in US—A—3 427 251. It has surprisingly been discovered that these problems can be overcome and relatively steady and unhindered permeation of active materials through body tissues can be achieved if certain quaternary ammonium compounds are used in which the anionic group represents a compound containing a long chain alkyl group, one or more ethylene-oxide residues and either a carboxylic or phosphoric group.

SUMMARY OF THE INVENTION

This invention relates to complexes which are useful as transdermal carriers of pharmaceutical active ingredients. The complexes enhance permeation through skin membranes of active pharmaceutical or cosmetic ingredients combined with them.

DETAILED DESCRIPTION OF THE INVENTION

The complexes of the invention have a cationic component of the formula

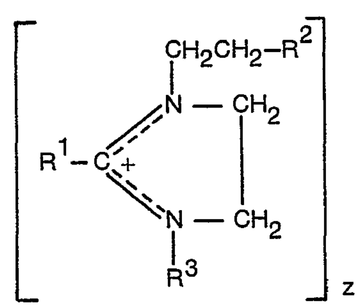

or

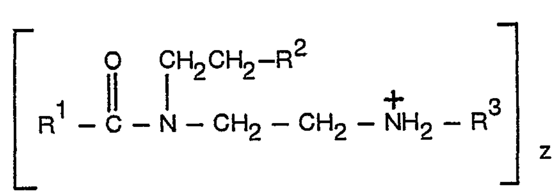

wherein:

$R^1$ is $C_6$—$C_{22}$ alkyl,

$R^2$ is a member of the group consisting of —$R^4$—$CH_2$—$CH_2$—COOH, —$R^4$—CH=CH—COOH and —$OPO_3H_2$, wherein $R^4$ is —NH—, —NH—CO—, or —O—CO—, and

$R^3$ is hydrogen, $—CH_2—COOH$ or $—CH_2CH_2—COOH$; and an anionic component which is either a carboxylate having the formula

$$^{-}OOC—CH_2—(O—CH_2—CH_2)_x—OC_nH_{2n+1}$$

or a phosphate having the formula

$$^{=}O_3PO—CH_2—CH_2—(OCH_2—CH_2)_x—OC_nH_{2n+1}$$

wherein:

n is 8 to 22 and x is 1 to 10; z is 1 when the anionic component is a carboxylate and z is 2 when the anionic component is a phosphate.

Preferred complexes of the invention are those having the above cationic component, wherein $R^1$ is $C_{12}H_{25}$ or, less preferably, the alkyl residues of caprylic, palmitic, stearic or behenic acid, $R^2$ is $—NH—CH_2—CH_2COOH$ or, less preferably, one of the above other groups, and $R^3$ is $—CH_2—CH_2—COOH$ or, less preferably, one of the other defined groups, complexed with the above anionic components wherein x is 5 and n is 12.

Complexes of the invention may be prepared by treating alkali metal salts of an imidazoline with a carboxylic or phosphoric acid in aqueous solution, said reactants having structures corresponding to those defined above for the cationic and anionic components. Stoichiometric amounts of the reactants may simply be stirred at 20—80°C, preferably 40°C for about 15 minutes, to form the complex. Complex formation has been verified by high voltage electrophoresis and column chromatography.

Imidazoline reactants wherein $R^3$ denotes $—CH_2—COOH$ or $—CH_2—CH_2—COOH$ and which are useful to form certain of the complexes of the invention may be obtained by the following exemplary reaction sequence: a) Reacting a carboxylic acid with a di- or triamine to obtain compound (A):

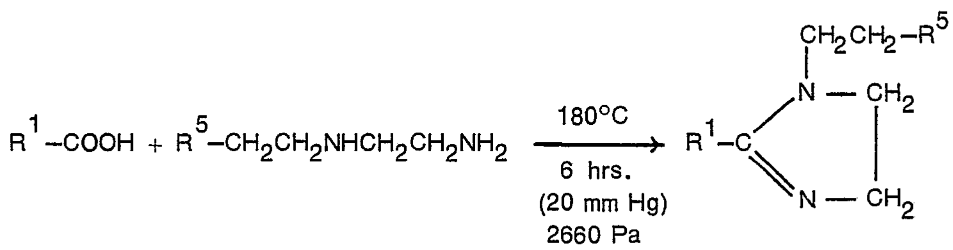

(A)

wherein $R^5$ is an hydroxyl, chloride or amino group and $R^1$ is as defined above. b) The resulting ring compound (A) is then reacted with acrylic or chloroacetic acid at 120°C for 4 hours to provide the corresponding substituent $R^3$ of compound (B):

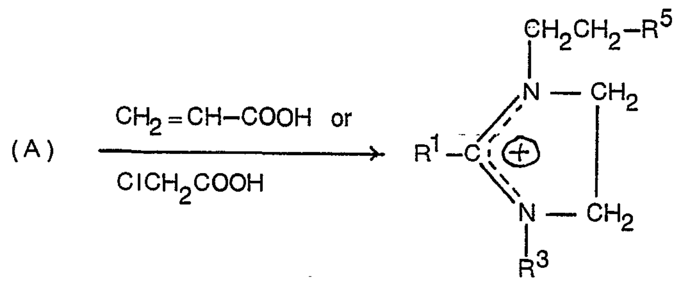

(B)

whereby $R^3 = -CH_2-CH_2-COO^-$ or $-CH_2-COO^-$, respectively and

c) The substiuent $R^5$ of (B) is converted into $R^2$ residues as follows:

(1) by dissolving compound (B) with $R^5 = —NH_2$ or $—OH$ in a solvent, such as chloroform or methylene chloride, and adding with stirring at room temperature acrylic acid or a suitable anhydride; or (2) by dissolving compound (B) with $R^5 = —Cl$ in aqueous solution and reacting with an appropriate amino acid at 50—80°C for one hour; or (3) by stirring compound (B) with $R^5 = —OH$ into polyphosphoric acid at 60°C for 1 hour.

It is pointed out that in the imidazoline reactants obtained in this way, the residues $—CH_2—CH_2—R^2$ and $R^3$ are attached to different N atoms of imidazoline ring.

The anion reactants are available commercially or may be formed by conventional techniques.

Active cosmetic or pharmaceutical ingredients can be combined with the complexes of the invention to thereby facilitate transport of the active ingredients through tissue membranes.

The substituents of the cationic component of the complexes of the invention are able to be metabolized into physiologically acceptable materials. This is believed to account, in part, for the suitability of these materials as skin transport vehicles. It is further postulated that the complexes of the invention are effective cell membrane transport vehicles due to their amphoteric resemblance to lipids and membrane proteins responsible for substance transport. Moreover the complexes appear to have relatively little or at least delayed denaturing effects on the membrane proteins and do not appear to change the protein configuration or solubilize the lipid layer of the cell membranes. It is believed that the complexes of the invention have minimal adverse effects on cell metabolism and, more specifically, the membrane transport system necessary therefor, due to these characteristics.

Where an active ingredient is strongly ionic or contains substituents which produce adverse effects on cell membranes, it may be rendered physiologically more compatible and transport thereof can be further enhanced if the active ingredient is used in the form of a salt with glutamic acid, aspartic acid, lysine or arginine, which salt is combined with a complex of the invention. Such salts mask the physiologically adverse effects of the active ingredient.

The complexes along with effective amounts of the active ingredients are formulated as a bath, cream or other composition for topical application or into a composition for oral administration. Such formulations generally are water based. In addition to the water base, carrier complex and active ingredient, other materials commonly employed in topical or oral pharmaceutical or cosmetic compositions may be included in the formulations. For example, preservatives, antimicrobials, foaming agents or the like can be employed. Surfactants, particularly sulfate surfactants, which inhibit membrane transport, should be avoided.

The following examples are illustrative of the invention.

Example 1

A preferred formulation for use in the application of cosmetic ingredients in a bath contains a complex having the structure

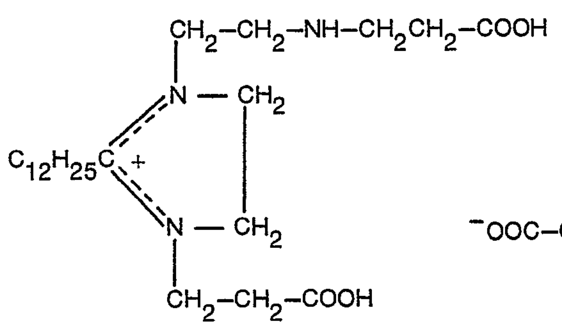

$^{-}OOC-CH_2-(O-CH_2-CH_2)_5-O-C_{12}H_{25}$

This complex is preferably applied in a composition containing:

| | |
|---|---|
| Complex | 15 parts |
| Cocofattyacid diglycolamide | 3 parts |
| Stearyl alcohol decaoxyethylate | 9 parts |
| Isopropanol | 5 parts |
| 4-Hydroxybenzoic acid methylester | 0.2 parts |
| 4-Hydroxybenzoic acid propylester | 0.1 part |

and sufficient active ingredient and water to bring the total composition to 100 parts.

Example 2

A preferred complex for use in a cream formulation has the structure

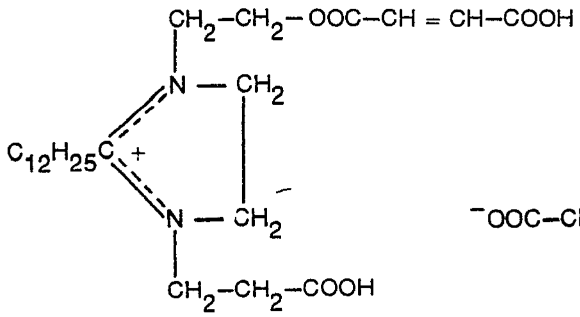

$^{-}OOC{-}CH_2{-}(O{-}CH_2{-}CH_2)_5{-}O{-}C_{12}H_{25}$

In addition, the cream preferably contains a salt of nicotinic acid and the imidazolin group of the complex employed in Example 1 to induce hyperemia in the skin to which the cream is applied. The composition of a cream preferably is as follows:

| | |
|---|---|
| Complex | 5% |
| Glyceryltricocoate | 38.5% |
| Glyceryltricaprylate | 5% |
| Vitamineoil (germ oils) | 2.5% |
| Lanolin | 1% |
| Nicotinic salt | 1.5% |
| Glycerol | 4% |
| Ascorbic acid | 3% |
| 4-Hydroxybenzoic acid methylester | 2% |
| 4-Hydroxybenzoic acid propylester | 1% |

and sufficient active ingredient and water to yield 100%.

Example 3

A formulation for oral administration preferably contains a complex of the structure

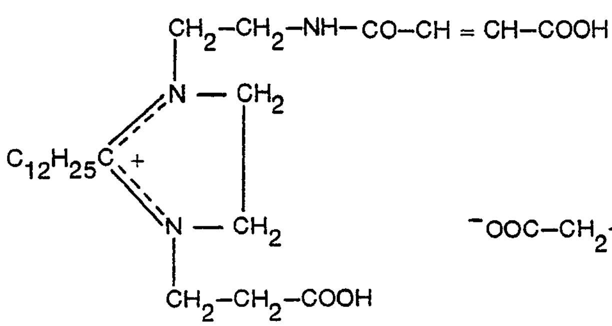

$^{-}OOC{-}CH_2{-}(OCH_2{-}CH_2)_5{-}OC_{12}H_{25}$

The complex is added to a formulation of the following composition:

| | |
|---|---|
| Complex | 0.2% |
| Phosphatidylcholin | 0.5% |
| Taurocholic acid Na salt | 1.9% |
| Vitamin oil (germ oil) | 12.5% |
| 4-Hydroxybenzoic acid methylester | 0.2% |
| 4-Hydroxybenzoic acid propylester | 0.01% |

and active ingredient and water sufficient to total 100%.

**Claims**

1. A complex consisting of
a) a cationic component having the formula

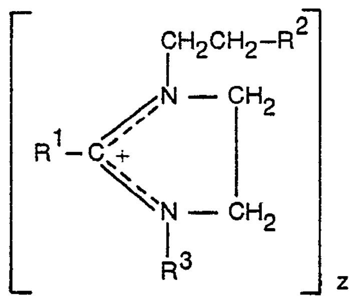

or

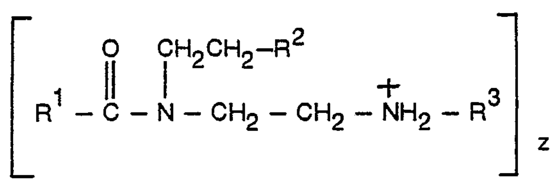

wherein:

$R^1$ is $C_6$—$C_{22}$ alkyl,

$R^2$ is —$R^4$—$CH_2$—$CH_2$—COOH, —$R^4$—CH=CH—COOH and —$OPO_3H_2$, wherein $R^4$ is —NH—, —NH—CO—, or —O—CO—, and

$R^3$ is hydrogen, —$CH_2$—COOH or —$CH_2$—$CH_2$—COOH; and

b) an anionic component which is either a carboxylate ion having the formula

$$^{-}OOC—CH_2—(O—CH_2—CH_2)_x—OC_nH_{2n+1}$$

or a phosphate ion having the formula

$$^{=}O_3PO—CH_2—CH_2—(OCH_2—CH_2)_x—OC_nH_{2n+1}$$

wherein:

n is 8 to 22 and x is 1 to 10; and z is 1 when the anionic component is a carboxylate and z is 2 when the anionic component is a phosphate.

2. A complex of claim 1 wherein $R^1$ is —$C_{12}H_{25}$, $R^3$ is —$CH_2CH_2COOH$, x is 5 and n is 12.
3. A complex of claim 2 wherein $R^2$ is —NH—$CH_2$—$CH_2$—COOH.
4. A complex of claim 2 wherein $R^2$ is —OOC—CH=CH—COOH.
5. A complex of claim 2 wherein $R^2$ is —NH—CO—CH=CH—COOH.

6. An aqueous base composition comprising a complex of claim 1 and an active pharmaceutical or cosmetic ingredient.

7. A bath formulation comprising the complex of claim 3 and an effective amount of an active pharmaceutical or cosmetic ingredient.

8. A cream formulation comprising the complex of claim 4 and an effective amount of an active pharmaceutical or cosmetic ingredient.

9. An oral formulation comprising the complex of claim 5 and an effective amount of an active pharmaceutical or cosmetic ingredient.

10. The formulations of claims 7, 8 or 9, comprising the pharmaceutical or cosmetic ingredient in the form of a salt with glutamic acid, aspartic acid, lysine or arginine.

**Patentansprüche**

1. Ein Komplex, bestehend aus

a) einer kationischen Komponente der Formel

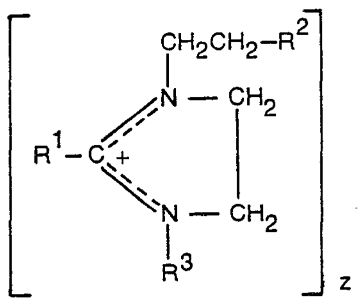

oder

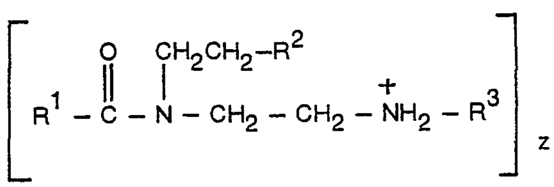

worin

$R^1$ $C_6$—$C_{22}$ Alkyl bedeutet,

$R^2$—$R^4$—$CH_2$—$CH_2$—COOH, —$R^4$—CH=CH—COOH und —$OPO_3H_2$ bedeutet, worin $R^4$ —NH—, —NH—CO— oder —O—CO— ist, und

$R^3$ Wasserstoff, —$CH_2$—COOH oder —$CH_2$—$CH_2$—COOH ist; und

b) einer anionischen Komponente, welche entweder ein Carboxylation der Formel

$$^{-}OOC—CH_2—(O—CH_2—CH_2)_x—OC_nH_{2n+1}$$

oder ein Phosphation der Formel

$$^{=}O_3PO—CH_2—CH_2—(OCH_2—CH_2)_x—OC_nH_{2n+1}$$

ist, worin

n 8 bis 22 ist, und x 1—10 ist; und z 1 ist, wenn die anionische Komponente ein Carboxylat ist, und z 2 ist, wenn die anionische Komponente ein Phosphat ist.

2. Ein Komplex nach Anspruch 1, dadurch gekennzeichnet, dass $R^1$ —$C_{12}H_{25}$ ist, $R^3$ —$CH_2CH_2COOH$ bedeutet, x 5 ist, und n 12 ist.

3. Ein Komplex nach Anspruch 2, dadurch gekennzeichnet, dass $R^2$ —NH—$CH_2$—$CH_2COOH$ ist.

4. Ein Komplex nach Anspruch 2, dadurch gekennzeichnet, dass $R^2$ —OOC—CH=CH—COOH ist.

5. Ein Komplex nach Anspruch 2, dadurch gekennzeichnet, dass $R^2$ —NH—CO—CH—COOH ist.

6. Eine wässrige Basiszusammensetzung, enthaltend einen Komplex nach Anspruch 1 und einen aktiven pharmazeutischen oder kosmetischen Bestandteil.

7. Eine Badformulierung, enthaltend den Komplex nach Anspruch 3 und eine wirksame Menge eines aktiven pharmazeutischen oder kosmetischen Bestandteils.

8. Eine Crèmeformulierung, enthaltend den Komplex nach Anspruch 4 und eine wirksame Menge eines aktiven pharmazeutischen oder kosmetischen Bestandteils.

9. Eine orale Formulierung, enthaltend den Komplex nach Anspruch 5 und eine wirksame Menge eines aktiven pharmazeutischen oder kosmetischen Bestandteils.

10. Formulierungen nach den Ansprüchen 7, 8 oder 9, enthaltend den pharmazeutischen oder kosmetischen Bestandteil in der Form eines Salzes mit Glutaminsäure, Aspartinsäure, Lysin oder Arginin.

**Revendications**

1. Complexe constitué de

a) un composant cationique répondant à la formule qui suit:

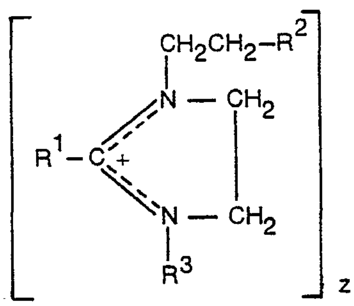

ou

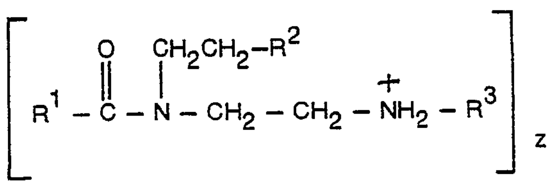

dans laquelle:

$R^1$ représente un radical alkyle en $C_6$—$C_{22}$;

$R^2$ représente le radical —$R^4$—$CH_2$—$CH_2$—COOH, —$R^4$—CH=CH—COOH ou $OPO_3H_2$, où $R^4$ représente le radical —NH—, —NH—CO— ou —O—CO—, et

$R^3$ représente un atome d'hydrogène, le radical —$CH_2$—COOH ou —$CH_2$—$CH_2$—COOH; et

b) un composant anionique qui est soit un ion carboxylate répondant à la formule

$$^{-}OOC—CH_2—(O—CH_2—CH_2)_x—OC_nH_{2n+1}$$

soit un ion phosphate répondant à la formule

$$^{=}O_3PO—CH_2—CH_2—(OCH_2—CH_2)_x—OC_nH_{2n+1}$$

dans laquelles:

n représente un nombre dont la valeur varie de 8 à 22 et X représente un nombre dont la valeur varie de 1 à 10; et z est égal à 1 lorsque le composant anionique est un carboxylate et z est égal à 2 lorsque le composant anionique est un phosphate.

2. Complexe suivant la revendication 1, caractérisé en ce que $R^1$ représente le radical —$C_{12}H_{25}$, $R^3$ représente le radical —$CH_2CH_2COOH$, x est égal à 5 et n est égal à 12.

3. Complexe suivant la revendication 2, caractérisé en ce que $R^2$ représente le radical —NH—$CH_2$—$CH_2$—COOH.

4. Complexe suivant la revendication 2, caractérisé en ce que $R^2$ représente le radical —OOCH—CH=CH—COOH.

5. Complexe suivant la revendication 2, caractérisé en ce que $R^2$ représente le radical —NH—CO—CH=CH—COOH.

6. Composition de base aqueuse comprenant un complexe suivant la revendication 1 et un ingrédient cosmétique ou pharmaceutique actif.

7. Composition de bain comprenant le complexe sivant la revendication 3 et une quantité efficace d'un ingrédient cosmétique ou pharmaceutique actif.

8. Composition de crème comprenant la complexe suivant la revendication 4 et une quantité efficace d'un ingrédient cosmétique ou pharmaceutique actif.

9. Composition à administrer par la voie orale, comprenant le complexe suivant la revendication 5 et une quantité efficace d'un ingrédient cosmétique ou pharmaceutique actif.

10. Compositions suivant les revendications 7, 8 ou 9, comprenant l'ingrédient cosmétique ou pharmaceutique sous la forme d'un sel avec l'acide glutamique, l'acide aspartique, la lysine ou l'arginine.